# EUROPEAN PATENT APPLICATION

(11) **EP 4 006 140 A1**
(43) Date of publication of application: **01.06.2022**
(21) Application number: 20383032.8
(22) Date of filing: 27.11.2020
(51) Int. Cl.: C12N 1/18, C12G 1/022, C12R 1/865

(54) **A SACCHAROMYCES CEREVISIAE STRAIN AND ITS USE FOR PRODUCTION OF REDUCED-ALCOHOL WINE**

(71) Applicant: Consejo Superior De Investigaciones Científicas (CSIC), 28006 Madrid (ES)
(72) Inventor: GONZÁLEZ GARCÍA, Ramón, 26080 Logroño (La Rioja) (ES); MORALES CALVO, Maria Pilar, 26080 Logroño (La Rioja) (ES); TRONCHONI LEÓN, Jordi, 26080 Logroño (La Rioja) (ES)
(74) Representative: Pons

(57) **Abstract**

The present invention relates to a novel *Saccharomyces cerevisiae* strain which presents low acetic acid yield under aerobic fermentation processes. This feature makes it especially useful for reducing the ethanol content of wines, while avoiding excess volatile acidity associated to other isolates of the same species, by providing sufficient oxygen during alcoholic fermentation. In contrast to strains from other yeast species, this strain is capable of driving wine fermentation to dryness. In this way the process is performed with the inoculation of a single *S*. *cerevisiae* yeast strain. The present invention also relates to the use and method for fermented beverage production, particularly production of quality wines with reduced ethanol content.

## Description

The present invention relates to a novel *Saccharomyces cerevisiae* strain which presents low acetic acid yield under aerobic fermentation processes reducing the ethanol content of wine. The invention belongs to the technical field of microbiology, particularly food microbiology.

### BACKGROUND ART

One of the main concerns of the wine industry in the past decades is the steady increase in the alcohol level of wines, which is directly related to the sugar content of the grapes at harvest. One of the main factors driving this problem is the imbalance between the technological, phenolic and aromatic maturity of the grapes due to global climate change, along with consumer preferences for well-structured, full-bodied wines with optimal phenolic maturity (*Mira de Orduña, 2010*).

Rising ethanol levels are detrimental for the wine industry in several respects, including public health concerns, a negative impact on the sensory quality of wines, and a growing tax burden in many countries. Researchers are working on different strategies to reduce the ethanol content of wine, spanning from selection of plant material to wine dealcoholisation, and including vineyard and fermentation management. Microbiological approaches are a relevant part of the toolkit currently under consideration; mainly focusing on reducing alcohol yield during fermentation.

*Saccharomyces cerevisiae* is widely regarded as the best adapted yeast species for wine fermentation; it is the main species that drives the process in non-inoculated fermentations, and for many decades it was almost the only yeast species marketed as a wine starter culture. Phenotypic variability in ethanol yield for this species is low *(Palacios A., et al 2007; Camarasa C., et al 2011*); probably due to its high specialization for alcoholic fermentation.

Biotechnological strategies based on S. *cerevisiae* to lower ethanol yield focus on modifying metabolic carbon flux of strains already in use. The objective is diverting sugar carbon to the production of other metabolites, mainly through metabolic engineering, but also by adaptive laboratory evolution (*reviewed in Tilloy et al 2015*).

The strict regulation of genetically modified organisms (GMOs) for food and beverages applications is not the only constraint to the use of genetic engineering in this context. Indeed, the overproduction of undesired metabolites in many of the recombinant strains obtained so far would preclude any practical use of such yeast strains in winemaking. Adaptive laboratory evolution holds greater application potential and has been successfully employed to redirect metabolic flux to glycerol production in industrial strains of *S*. *cerevisiae* (*Tilloy et al 2014*).

One way to circumvent the metabolic rigidity of S. *cerevisiae* in terms of ethanol yield is using strains of other yeast species. In order to ensure complete fermentation of grape must, these new species should typically be used in combination with S. *cerevisiae,* either in a simultaneous or sequential inoculation (*Ciani M., et al 2016*). Several authors have indeed explored inter and intraspecific variability in ethanol yields among different species of wine yeasts (*Gobbi M., et al 2014, Contreras A., et al 2014; Castellari L., et al* 1994; *Arroyo-Lopez F.N., et al 2010*).

In order to further divert the yeast metabolism away from ethanol production, it was proposed a few years ago to switch to sugar respiration. The proposed procedure involved using aerobic conditions during the first days of fermentation, to provide the required oxygen, and relied on *non-Saccharomyces* strains to overcome the Crabtree positive feature of S. *cerevisiae* (*Gonzalez R., et al 2013*). Initial strain screening and laboratory trials showed that, despite the Crabtree effect, the ethanol yield of S. *cerevisiae* was considerably lower under aerobic conditions than under standard wine fermentation conditions (*Morales P., et al 2015*); which would open the possibility of using S. *cerevisiae* for alcohol level reduction by respiration. However, their high acetic acid yield under aerobic conditions rendered these strains of S. *cerevisiae* unsuitable for the process. The use of Crabtree negative *non-Saccharomyces* yeasts is interesting, but requires optimization of yeast strain production, inoculation strategies, yeast nutrient management, and other process parameters.

In previous reports, it was shown that some genetic modifications of S. *cerevisiae* allowed to alleviate the problem of excess volatile acidity under aerobic conditions, making these strains suitable for the reduction of alcoholic strength by respiration (*Curiel et al 2016*).

Due to the strict regulation of genetically modified organisms (GMOs) for food and beverages applications and the increase of acetic acid yield under aerobic conditions in a simultaneous inoculation with S. *cerevisiae* and *non-Saccharomyces* strains, it is necessary to develop alternative procedures with acceptable acetic acid yield under aerobic fermentation processes in order to reduce the ethanol content of wine.

### SUMMARY OF THE INVENTION

The present invention relates to a novel *Saccharomyces cerevisiae* strain which presents low acetic acid yield under aerobic fermentation processes reducing the ethanol content of wine. This yeast can be employed in winemaking without any regulatory restrictions, resulting in better process simplicity and robustness (single inoculation step and no concerns about sulphite tolerances by *non-Saccharomyces* strains, or other problems affecting their competitive performance in fermenting grape must).

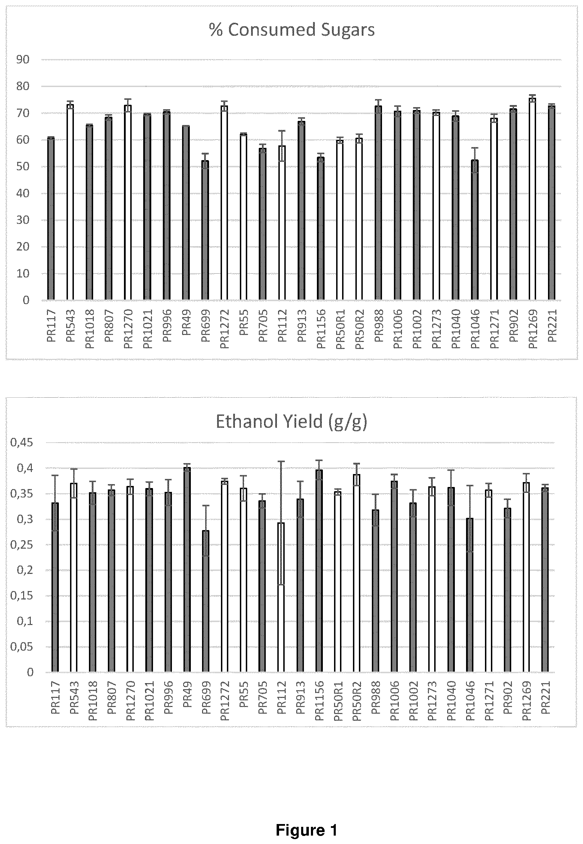

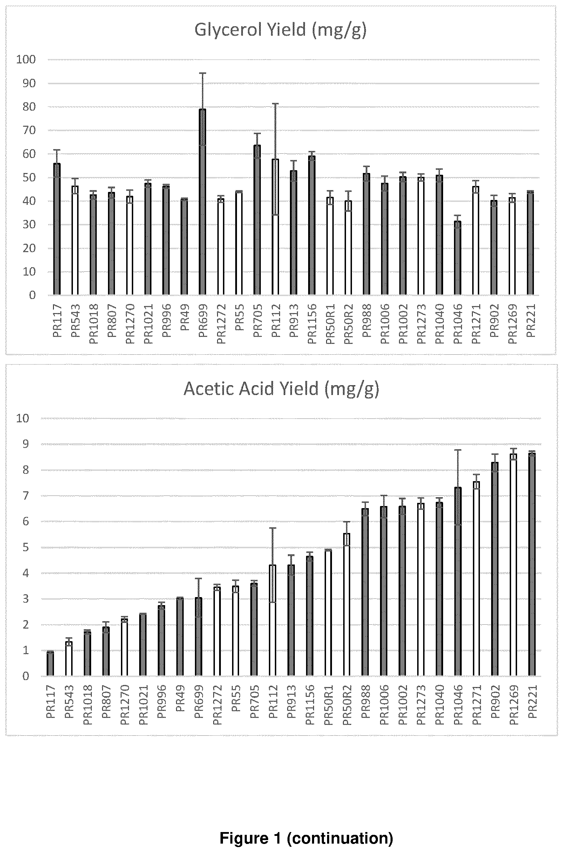

The inventors cultivated twenty-six S. *cerevisiae* strains in natural grape must under aerobic conditions and analyzed the sugar consumption, the ethanol yield, the glycerol yield and acetic acid yield. The inventors selected the S. *cerevisiae* strains PR117, PR543 and PR1018 with low acetic acid yield (Figure 1) for further characterization, and strain PR50 as an example of a representative S. *cerevisiae* strain showing elevated acetic acid production under aerobic conditions.

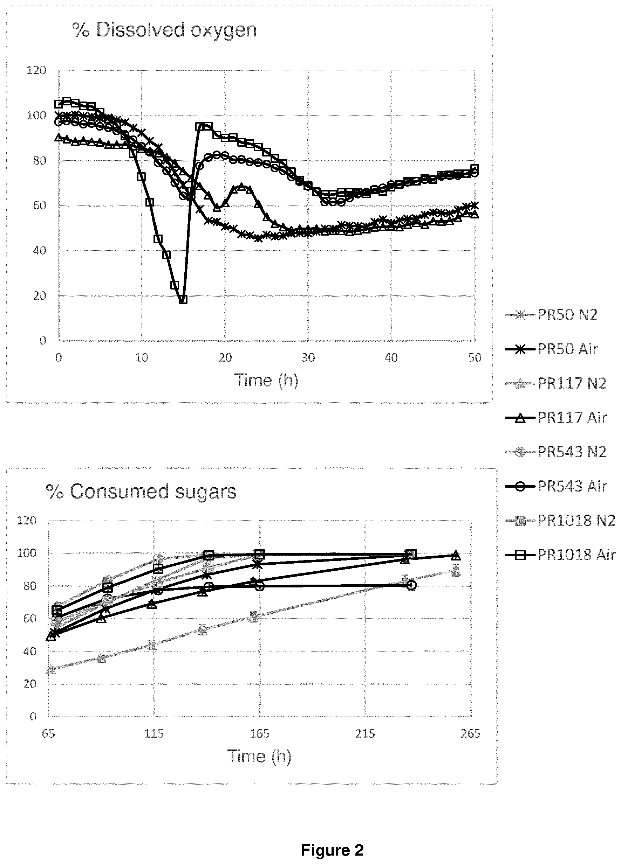

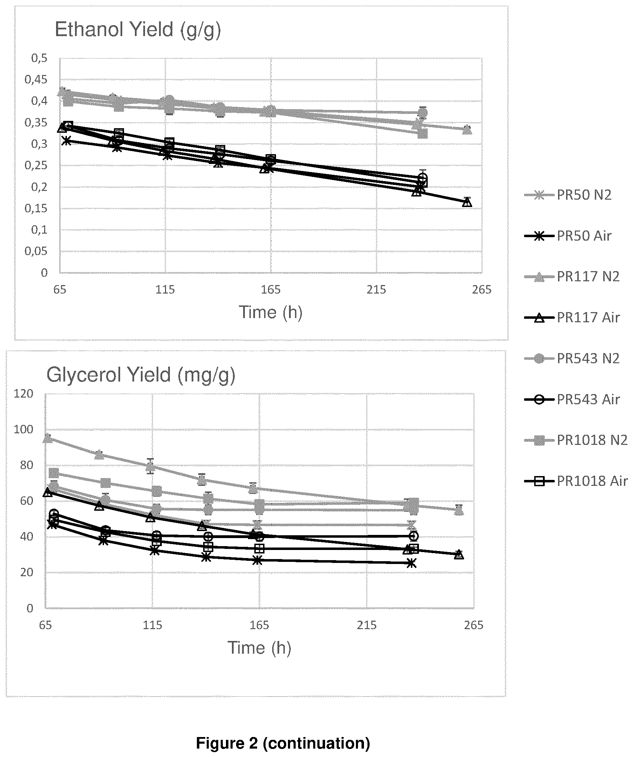

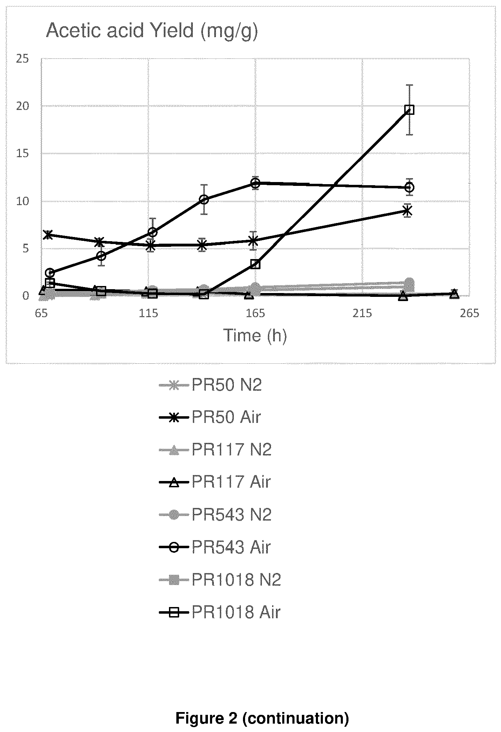

The inventors carried out fermentation experiments in bioreactors with a constant gas flow (N₂ or air) for comparing the ethanol and acetic acid yields between aerobic and anaerobic conditions. The ethanol yield of all four strains was consistently lower under aerobic than under anaerobic conditions. For the same growth condition, all strains showed very similar values (ethanol yield in aerobiosis was 0.27-0.30g/g, Table 2) (Figure 2).

Strain S. *cerevisiae* PR1018 (with deposit number CECT 13195) had a remarkably low acetic acid yield during the first days of aerobic fermentation (Figure 2). The production of acetaldehyde and its acetal derivatives was higher under aerobic conditions, but significantly lower for strain S. *cerevisiae* PR1018 (CECT 13195) than for the other three strains (Figure 3). The volatile profile of PR1018 under aerobic conditions is the one falling closer to anaerobic conditions among the yeast strains tested (Figure 4).

The strain *S*. *cerevisiae* PR1018 (CECT 13195) shows the features that make it interesting candidate for low alcohol winemaking under aerobic conditions minimizing the impact of aeration on the sensory features of wines.

A first aspect of the present invention related to a *Saccharomyces cerevisiae* strain with deposit number CECT 13195, hereinafter the "strain of the invention" or "CECT 13195 strain" or "PR1018 strain".

Said strain was deposited by Consejo Superior de Investigaciones Científicas (CSIC) on 27 October 2020 under the Budapest Treaty with the Spanish Type Culture Collection (CECT) and assigned deposit number CECT13195. The address of said international deposit Authority is Edificio 3 CUE, Parc Científic Universitat de Valencia, Catedrático Agustín Escardino 9, 46980 Paterna (Valencia), Spain.

*Saccharomyces cerevisiae* is a species of yeast (single-celled fungus microorganisms). The species has been instrumental in winemaking, baking, and brewing since ancient times. It is the microorganism behind the most common type of fermentation. It belongs to superkingdom, *Eukaryota;* clade, *Opisthokonta*; kingdom, *Fungi;* subkingdom, *Dikarya;* phylum, *Ascomycota;* clade, *Saccharomyceta;* subphylum, *Saccharomycotina*; class, *Saccharomycetes;* order, *Saccharomycetales*; family, *Saccharomycetaceae;* and genus, *Saccharomyces.* The novel S. *cerevisiae* CECT 13195 (*S. cerevisiae* PR1018) has been isolated from grape must, presents low acetic acid yield under aerobic fermentation processes, enabling its use to reduce the ethanol content of wine.

Another aspect of the present invention relates to a strain derived from the S. *cerevisiae* CECT 13195, wherein said strain maintains or improves the capabilities described throughout the present invention. The microorganism derived can be naturally occurring or produced intentionally by mutagenesis methods known in the prior art such as, but not limited to, growth of the original microorganism in the presence of mutagenic agents or stressors, or directed genetic engineering of specific genes. According to a preferred embodiment, the strain derived from the S. *cerevisiae* CECT 13195 strain is a genetically modified mutant. The terms "mutant strain" or "derived strain" can be used interchangeably.

The *S*. *cerevisiae* CECT 13195 strain or any mutant or derivative thereof may be used in any way to exert the effects described, for example, according to a preferred embodiment of the present invention, the *S*. *cerevisiae* CECT 13195 strain is formed of viable cells (cultivable or uncultivable).

Hereinafter, any of the strains of the previously described species *S*. *cerevisiae* (*S. cerevisiae* CECT 13195 strain or any mutant or derivative thereof) may be referred to as the "strain of the present invention" or the "strain of the invention."

Another aspect of the present invention relates to a composition, hereinafter the "composition of the invention", comprising the strain of the invention.

The composition, generally defined, is a set of components which is formed at least by the strain of the invention at any concentration.

According to the invention, the previous composition may further comprise at least one additional microorganism other than the strain of the invention. In a preferred embodiment of the invention, the additional microorganism is a *Saccharomyces* strain and/or *Non-Saccharomyces* strain. For example, but not limited to, the additional microorganism that may form part of said composition is selected from: *Metschnikowia pulcherrima, Torulaspora delbrueckii*, *Hanseniaspora uvarum, Lachancea thermotolerans*, *Candida sake, Kluyveromyces lactis*, *Pichia fermentans, Starmerella bombicola*, *Starmerella bacillaris*.

Optionally, the composition according to any of those defined above may further comprise at least one additive. Thus, in a preferred embodiment the composition of the invention further comprises at least one additive.

The term "additive" relates to a compound, substance, solution, product which can improve, enhance or supplement the activity of a strain of the species *S. cerevisiae* and preferably the *S. cerevisiae* CECT 13195 strain object of the invention, or which is added to fermented beverages by a *S. cerevisiae* strain, preferably the *S. cerevisiae* CECT 13195 strain object of the invention, in order to improve or preserve the taste, appearance, freshness and quality, maintaining its chemical and organoleptic properties. Examples of additive include, without limitation to, food ingredients or components (poly-unsaturated fatty acids, conjugated linoleic acid, prebiotics, fibre, guar gum, glucomannan, chitosan, copper, calcium, etc.), plants or plant extracts or components (polyphenols, argan oil, clarification proteins), and chemicals or enzymes (sulfiting agents, lysozyme, glycosidases, pectinases, hemicellulases).

Fermented beverages are produced following the fermentation of sugars by yeasts, mainly strains of the species *Saccharomyces cerevisiae.* In the presence of sugars, together with other essential nutrients such as amino acids, minerals and vitamins, S. *cerevisiae* will conduct fermentative metabolism to ethanol and carbon dioxide. As described previously, the strain *S. cerevisiae* PR1018 (CECT 13195) shows the features that make it interesting candidate for low alcohol beverages making under aerobic conditions minimizing the impact of aeration on the sensory features of beverages.

Thus, another aspect of the invention relates to the use of the strain of the invention and/or the composition of the invention or any previously defined combination thereof, hereinafter the "use of the invention", for fermented beverages production.

The term "fermented beverages" or "alcoholic fermented beverages", used interchangeably in the present invention, refers to beverages or drinks produced by or which have undergone, a process of fermentation of sugars by yeasts, mainly by *Saccharomyces cerevisiae.* In the presence of sugars *S. cerevisiae* will conduct fermentative metabolism to ethanol. Examples of fermented beverages (but not spirituous or distilled liquors) include, without limitation to, beer, wine, vinegar, champagne, cava, sake and cider.

In a preferred embodiment of the use of the invention, the fermented beverage is selected of the list consisting of: wine, beer, champagne, cava, sake and cider.

In other preferred embodiment of the use of the invention, the fermented beverage is wine.

Fermented beverages are produced following the fermentation of sugars to ethanol by yeasts, mainly strains of the species *Saccharomyces cerevisiae.* As described previously, the strain *S. cerevisiae* PR1018 (CECT 13195) produces low alcohol beverages making under aerobic conditions.

Thus, other aspect of the invention relates to a method for fermented beverages production, hereinafter the "method of the invention", which comprises:
(i) inserting the strain of the invention and/or the composition of the invention in a sugar-containing solution, and
(ii) incubating the sugar-containing solution obtained in (i) at a temperature between 8ºC and 35ºC during 2 to 10 days under aerobic conditions, obtaining a partially or fully fermented sugar-containing solution, wherein the sugar-containing solution of step (i) comprises a hexose content between 15-35% and the fermented sugar-containing solution obtained in step (ii) comprises a residual hexose content between 0 to 10%.

The term "fermented beverages" and the strain, the cellular constituents, metabolites, molecules secreted by the strain and the composition of the invention, which have been defined in previous paragraphs of the present document, apply in the same way to this aspect of the invention, as well as all its particular embodiments (alone or in combination).

The term "sugar-containing solution" used herein, refers to a juice, nectar, must, squeezed, solution or extract which comprises high sugar content, typically between 10 and 40%, from variety of sugar-containing materials like cereals, fruits and vegetable juices, tea, and milk. The amount of sugar in must indicates the amount of alcohol that could be produced if it is all fermented to alcohol, rather than left as residual sugar. For example, the fermented beverages obtained from different sources are beer from barley, kefir from milk, wine from grapes, cider from apples, sake from rice, mead from honey.

So, in a preferred embodiment of the method of the invention the fermented beverage is selected of the list consisting of: wine, beer, sparkling wine, sake and cider. In other preferred embodiment of the method of the invention the fermented beverage is wine.

Fermented beverages may be made from a variety of sugar-containing solutions like cereals, fruits and vegetable juices, tea, and milk, preferably cereals or fruit. Examples of cereals include, without limitation to, rice, oats, barley, wheat, rye, corn and sorghum. Examples of fruits include, without limitation to, grape, apple, pear, pineapple, orange, melon, cherry, strawberry, peach, raspberry, durian, blackberry, orange, pomegranate, kiwi, tangerine, pear, peach and apricot.

In other more preferred embodiment of the method of the invention, the sugar-containing solution is a must, particularly is grape must. In other even more preferred embodiment of the method of the invention, the must is white, rose, or red must or grape mass.

In other even more preferred embodiment of the method of the invention, the white must is of *Vitis vinifera* varieties, preferably of Malvasia, Viura, White Grenache, White Tempranillo, White Maturana, Turruntes, Chardonay, Sauvignon Blanc, Albariño, Gewürztraminer, Moscatel, Verdejo, Riesling, Viognier, Chenin Blanc, Xarello, Palomino, Airen, Godello, Pinot Grigio or Semillon.

In other even more preferred embodiment of the method of the invention, the red grape mass is of *Vitis vinifera* varieties, preferably of Tempranillo, Red Maturana, Mazuelo, Grenache, Graciano, Cabernet Sauvignon, Pinot Noir, Pinot Meunier, Merlot, Syrah, Sangiovese, Bobal, Monastrell, Cariñena, Nebbiolo, Tanat, Carménère, Malbec, Gamay, Mencía, or Cabernet franc.

As understood by a skilled person in the art, the sugar-containing solution or sugary starting materials may require preparation processes before fermentation process. Examples of these preparation processes include, without limitation to: enzymatic process, hydrolysis process, filtration process, squeezing process, pressing process, destemming, cold maceration, carbonic maceration, thermal treatment, nitrogen source correction, total acidity correction, deoxygenation, or macrooxygenation.

The sugar-containing solution of the step (i) method of the invention comprises a hexose content between 15-35%. The term "hexose content" used herein refers to the amount, quantity or proportion of a hexose, monosaccharide (simple sugar) with six carbon atoms, contained in the sugar-containing solution in step (i). Examples of hexose include, without limitation to, allose, altrose, glucose, fructose, mannose, gulose, idose, galactose, talose, psicose, fructose, sorbose, tagatose, their isomers or enantiomers and any combination thereof. In a preferred embodiment of the method of the invention, the hexose is an equimolar mixture of glucose and fructose.

The hexose content in step (i) of the method of the invention is between 15-35%. In a preferred embodiment of the method of the invention, the hexose content is between 20 to 30%, preferably 22 to 28%. In another preferred embodiment, the hexose content is 23%, 24%, 25%, 26% or 27%, preferably the hexose content is 25%.

In the method of the invention, after inserting the strain of the invention, and/or the composition of the invention in a sugar-containing solution, the must obtained is incubated at a temperature between 8ºC and 35 ºC during 2 to 10 days, under aerobic conditions.

In a preferred embodiment of the method of the invention, the temperature of step (ii) is between 15ºC and 35ºC. In other preferred embodiment of the method of the invention, the temperature is between 18ºC and 30ºC. In other more preferred embodiment of the method of the invention, the temperature is 18ºC, 19ºC, 20ºC, 21ºC, 22ºC, 23ºC, 24ºC, 25ºC, 26ºC, 27ºC, 28ºC, 29ºC or 30ºC, preferably the temperature is 25ºC.

In other preferred embodiment of the method of the invention, the incubation period of the step (ii) is during 3, 4, 5, 6, 7, 8 or 9, preferably is 5 days.

The term "aerobic conditions" used herein, refers to growth in an oxygenated environment, i.e. fermentation in the presence of an active supply of oxygen (or air). Examples of methods for obtaining the aerobic conditions or oxygen supply include, without limitation to, bubbling, gas flow, or pumping over.

In other preferred embodiment of the method of the invention, the aerobic conditions of step (ii) comprise a constant gas flow with compressed air at 1 to 20 VVH (volumes of air per volume of liquid per hour), preferably the air flow is 10 VVH.

In the method of the invention, after of the step (ii) incubating the strain and/or the composition of the invention in a sugar-containing solution under aerobic conditions, is obtained a partially or fully fermented sugar-containing solution.

The term "fermented sugar-containing solution" used herein, refers to a solution, juice, nectar, must, squeezed or extracted which is obtained in a fermentation process wherein the initial amount of sugar has been partially or fully fermented to alcohol. As understood by a skilled person in the art, the fermented sugar-containing solution may comprise residual sugar content, preferably residual hexose content.

In the method of the invention, the fermented sugar-containing solution obtained in step (ii) comprises a residual hexose content between 0 to 10%.

When, after incubating the sugar-containing solution, a partially fermented sugar-containing solution is obtained in step (ii), alternatively the method of the invention, comprises a step (iii) incubating the partially fermented sugar-containing solution obtained in (ii) under anaerobic conditions at a temperature between 8ºC and 35ºC, until sugar exhaustion. It is routine practice for a person skilled in the art to determine the sugar exhaustion in the solution.

The term "partially fermented sugar-containing solution" used herein, refers to a solution, juice, nectar, must, squeezed or extracted which comprises low sugar content (as residual sugar), which is obtained in a partial fermentation process wherein the initial amount of sugar has not been fully fermented to alcohol.

The temperature conditions have been defined in previous paragraphs of the present document, apply in the same way to this step (iii) of the method of the invention, as well as all its particular embodiments (alone or in combination).

The term "anaerobic conditions" used herein, refers to growth in a non-oxygenated environment, i.e. fermentation in the absence of an active supply of oxygen (or air). Examples of methods for obtaining the anaerobic conditions include, without limitation to, adjustable vat, nitrogen sparging or static incubation of large volumes.

By means of the method of the invention a fermented beverage with reduced ethanol content and low acetic acid yield under aerobic fermentation is obtained. So, other aspect of the invention is the fermented beverage with reduced ethanol content obtained by the method of the invention, hereinafter the "fermented beverage of the invention".

In a preferred embodiment the fermented beverage of the invention comprises an alcoholic or ethanol degree (% v/v) ranges between 1 to 15 %v/v, more preferably ranges between 3 to 12 %v/v. In another preferred embodiment the fermented beverage is 4%v/v, 4.5%v/v, 5%v/v, 5.5%v/v, 6%v/v, 6.5%v/v, 7%v/v, 7.5%v/v, 8%v/v, 8.5%v/v, 9%v/v, 9.5%v/v, 10%v/v, 10.5%v/v, 11%v/v, or 11.5%v/v.

The term "fermented beverages" and the method of the invention, which have been defined in previous paragraphs of the present document, apply in the same way to this aspect of the invention, as well as all its particular embodiments (alone or in combination).

In a preferred embodiment of the invention the fermented beverage with reduced ethanol content is selected of the list consisting of: wine, beer, sparkling wines, sake and cider. In other preferred embodiment of the invention the fermented beverage is wine with reduced ethanol content.

Throughout the description and claims the word "comprises" and its variants are not intended to exclude other technical features, additives, components or steps. For persons skilled in the art, other objects, advantages and features of the invention will become apparent partly from the description and partly from the practice of the invention. The following figures and examples are provided by way of illustration, and are not intended to be limiting of the present invention.

### DESCRIPTION OF THE DRAWINGS

**Figure 1****.** Measure of main fermentation parameters of cultures of *Saccharomyces cerevisiae* after 4 days of growth on sterilised natural must (sugars 220 g/L). Black bars, wild isolates; white bars, commercial strains.
**Figure 2****.** Measure of dissolved oxygen and main fermentation parameters of selected strains of *Saccharomyces cerevisiae* on sterilised natural must (sugars 220g/L) in bioreactors, in aerobiosis and in anaerobiosis. Codes for strains and aeration are included. All results shown are the average of three biological replicates.

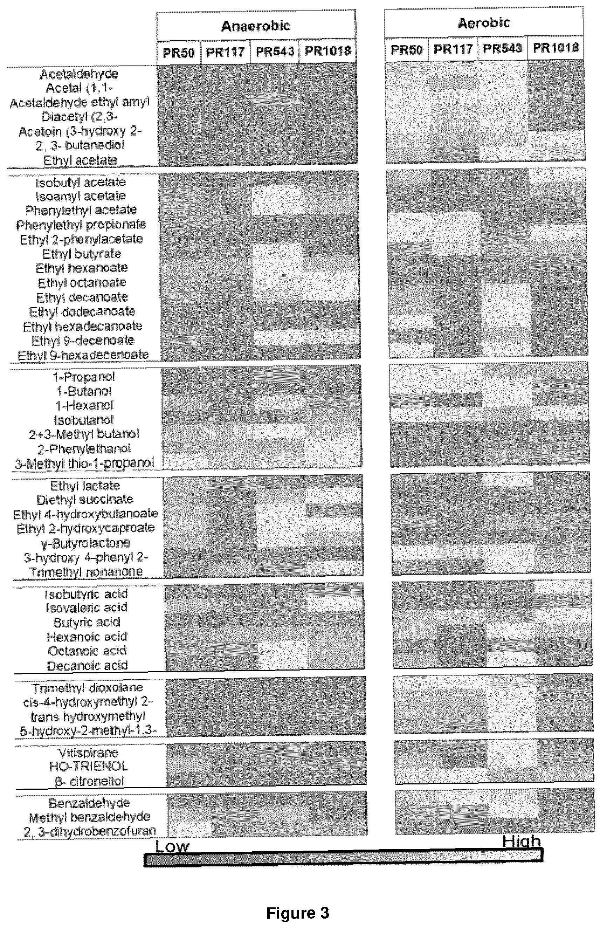

**Figure 3****.** Heatmap of yield of volatile compounds found in cultures in bioreactors. Greyscale is stablished for each compound between maximal and minimal values.

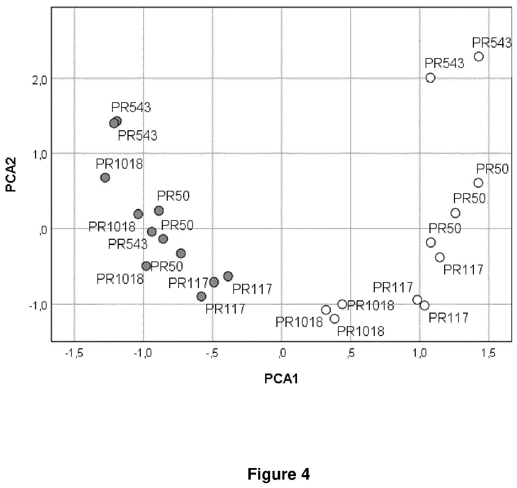

**Figure 4****.** Principal component analysis based on volatile compounds presented in Fig. 3 for S. *cerevisiae* strains under two different aeration conditions. Air conditions, white circles; nitrogen conditions, grey circles.

### EXAMPLES

### 1. MATERIALS AND METHODS

### 1.1. Yeast strains and media

Twenty-six *Saccharomyces cerevisiae* strains were used, nine of them are commercial wine yeast starters, and 17 of them are wild isolates from the PRICVV collection (PR collection of the *"Instituto de Ciencias de la Vid y del Vino*") (Table 1). Yeasts were maintained at 4°C on YPD plates (2% glucose, 2% peptone, 1% yeast extract and 2% agar), as well as in glycerol stocks at -80°C. Inocula were grown on YPD for 48h at 25°C, washed and resuspended in water. Natural white must from the 2019 harvest was kept frozen. The must contained 220g/L of sugar and 186mg/L yeast assimilable nitrogen. Enough volume for each experiment was thawed and pasteurized. Pasteurization consisted of heating to 105°C and then allowing it to cool down inside the closed autoclave.

**Table 1. Saccharomyces cerevisiae strains**

| **Code** | **Isolated from** |
|---|---|
| PR49 | Pedro Ximenes grapes |
| PR50 | Commercial wine starter |
| PR55 | Commercial wine starter |
| PR112 | Commercial wine starter |
| PR117 | Spontaneous "pris de mouse" |
| PR221 | Concentrated grape must |
| PR543 | Commercial wine starter |
| PR699 | Tempranillo juice |
| PR807 | White grape juice |
| PR902 | Red grape juice |
| PR913 | Red grape juice |
| PR988 | Tempranillo juice |
| PR996 | Cabernet Sauvignon juice |
| PR1002 | Cabernet Sauvignon juice |
| PR1006 | Tempranillo juice |
| PR1018 | Cabernet Sauvignon juice |
| PR1021 | Tempranillo juice |
| PR1040 | Tempranillo juice |
| PR1046 | Cabernet Sauvignon juice |
| PR1156 | Unknown (laboratory contaminant) |
| PR1269 | Commercial wine starter |
| PR1270 | Commercial wine starter |
| PR1271 | Commercial wine starter |
| PR1272 | Commercial wine starter |
| PR1273 | Commercial wine starter |

### 1.2. Aerobic fermentation of grape must

All experiments were performed in triplicate. For fermentation in shake flasks (100mL nominal volume) these were filled with 20mL pasteurized grape must, inoculated at 0.2 final OD600, capped with aluminium foil, and incubated at 25°C 180 rpm. After 4 days, samples were withdrawn, centrifuged, and supernatants were kept frozen for HPLC analysis.

For bioreactor assays, batch cultures were performed using *Applikon MiniBio* bioreactors (250mL nominal volume), equipped with Peltier refrigerated gas condensers. Bioreactors were filled with 150mL of pasteurized natural white grape must, and 200µL of antifoam 204 (Sigma-Aldrich, Spain). Temperature was set to 25°C and stirring to 1000 rpm. The cultures were sparged (with compressed air or pure nitrogen) at 25mL/min (10 VVH). Gas flow was controlled with MFC17 mass flow controllers (Aalborg Instruments and Controls, Inc., Orangeburg, NY), whose calibration was regularly verified with an electronic flowmeter (Agilent Technologies, Santa Clara, CA). Bioreactors were inoculated to 0.2 final OD600 with independent inocula prepared as described above. During the experiment, 1mL samples were collected at the designated times for HPLC analysis; as well as an additional 10mL final sample for the analysis of volatile compounds. Samples were centrifuged and supernatants were kept frozen for chromatographic analyses.

### 1.3. Determination of metabolite concentration

The concentration of glucose, fructose, glycerol, ethanol, and acetic acid was determined using a Surveyor Plus liquid chromatograph (Thermo Fisher Scientific, Waltham, MA) equipped with a refraction index and a photodiode array detector (Surveyor RI Plus and Surveyor PDA Plus, respectively) on a 300 × 7.7 mm PL Hi-Plex H⁺ (8 µm particle size) column (Agilent Technologies, Santa Clara, CA) and 4 x 3 mm ID Carbo-H guard (Phenomenex, Torrance, CA). The column was maintained at 50°C and 1.5mM H₂SO₄ was used as the mobile phase at a flow rate of 0.6mL/min. Prior to injection in duplicate, the samples were filtered through 0.22µm pore size nylon filters (Micron Analitica).

### 1.4. Analysis of volatile compounds

Samples for gas chromatography-mass spectrometry (GC-MS) analysis contained 1800µL of the original sample in a final volume of 2000µL, completed with ethanol and water to get the same amount of ethanol in all simples, 1g NaCl, and 20µL internal standard, in 20 mL flasks. Internal standard contained 1000 ppm each of 4-methyl 2-pentanol and heptanoic acid, and 100 ppm each of ethyl nonanoate and 1-nonanol, in water, prepared from 10000 ppm individual solutions in ethanol. Sample was preincubated for 10 min at 45ºC, followed by 30 min at 45ºC with 50/30µm DBV/CAR/PDMS SPME fiber (Stableflex, SUPELCO, Bellefonte, PA). Fiber was desorbed for 5 min at 250ºC.

GC-MS was carried out in a Thermo TRACE GC Ultra apparatus coupled to a Thermo ISQ mass detector, equipped with a Thermo TriPlus autosampler. Gas chromatography was carried in a Thermo Scientific fused-silica capillary column TG-WAXMS A (30m long; 0.25mm OD; 0.25µm film thickness). Chromatographic conditions were as follows: 5 min at 40ºC, 3ºC/min up to 200ºC, 15ºC/min up to 240ºC, 10 min at 240ºC. Helium was used as carrier gas at a flow rate of 1mL/min, operating in split mode (ratio 30). Total analysis time was 71 min. Detection was performed with the mass spectrometer operating in the Full Scan mode (dwell time 500ms), with 70eV ionization energy, and source and quadrupole temperatures of 250ºC. Detection was stopped during the time interval for ethanol elution. Peak identification was made by comparison of ion spectra with NIST mass spectral library. For each compound, including internal standards, the sum of the areas of the peaks of selected characteristic ions was obtained.

### 1.5. Statistical analysis

The levels and yield on substrate of the main fermentation metabolites and volatile compounds were compared by one-way analysis of variance. Comparison of means was carried by Tukey test. All analyses were performed using SPSS Statistics v.25 program (IBM, Armonk, NY, United States).

### 2. RESULTS

### 2.1. Aerobic growth of S. cerevisiae strains

Twenty-six *S. cerevisiae* strains were grown for 4 days in natural grape must under aerobic conditions, in triplicate. For operational reasons, the strains had to be distributed into two batches. Strain PR50 was included in both batches and was treated as different strains for the analyses (noted as PR50R1 and PR50R2 in the figures). Statistical analysis showed no differences between repetitions of PR50 for any of the parameters studied.

Sugar consumption varied between 52% and 75% of the original content. The four strains showing the lowest sugar consumption were wild isolates, whereas the four strains with the highest levels of sugars consumed were commercial strains.

The ethanol yield (g of ethanol produced per g of sugars consumed) determined under these experimental conditions ranged from 0.28 g/g for strain PR699 to 0.40 g/g for strain PR49 (Figure 1). The highest value is far below 0.45 g/g, a typical value for anaerobic conditions. This could be partly due to alcohol stripping, but also probably to yeast respiratory metabolism under aerobic conditions. Anyway, the ethanol yield found in this experiment involves a significant reduction in wine alcohol content compared to anaerobic conditions.

Glycerol yields ranged from 31 to 79 mg/g (Figure 1). Wild strain PR699 was significantly the highest glycerol producer. The difference in glycerol yield partly but not fully explain those in ethanol yields between the lowest and highest ethanol producing strains (PR699 and PR49, respectively). A relation of 2.5x was found between the highest and the lowest values of glycerol yield, similar to the variability found for glycerol production under anaerobic conditions.

The most variable parameter in this analysis was acetic acid yield, spanning almost one log unit, between 0.93 and 8.64mg/g, for strains PR117 and PR221 respectively (Figure 1). Interestingly, some of the strains show very low acetic acid yield under these experimental conditions, similar (or lower) to those typically found for commercial *S. cerevisiae* strains under anaerobic conditions. Among the three strains showing the lowest acetic acid yields, we found one commercial wine yeast strain PR543 and two wild isolates PR117 and PR1018.

### 2.2. Main fermentation parameters under aerobic and anaerobic conditions

The three strains with lower acetic acid production, one commercial wine yeast strain, PR543, and two wild isolates, PR117 and PR1018, were selected for further characterization. Strain PR50 was used as an example of a representative *S. cerevisiae* strain showing elevated acetic acid production under aerobic conditions. Fermentation experiments were carried out in bioreactors with a constant gas flow (N₂ or air). Using the same gas flow for aerobic and anaerobic conditions contributed to equalize ethanol stripping and allowed for better comparison of ethanol yields between aerobic and anaerobic conditions.

Dissolved oxygen in aerobic cultures decreased after inoculation for all four yeast strains (Figure 2). After reaching a minimal value (between 15 and 30 hours from the time of inoculation), the DO values gradually increased over time. However, each strain showed a distinctive pattern, which was consistent among replicates, in the evolution of DO during these aerobic fermentation experiments. Strain P1018 was the one showing a faster oxygen consumption in the initial stages of fermentation, with a sharp V-shape curve during the first day of culture, after which its behaviour was closer to the other strains. Sustained oxygen consumption confirms that all four strains were deploying respiro-fermentative metabolism under aerobic conditions.

Respiro-fermentative metabolism under aerobic conditions was also supported by comparing ethanol yields between aerobic and anaerobic conditions for each yeast strain (Figure 2). The ethanol yield of all four strains was consistently lower under aerobic than under anaerobic conditions. For the same growth condition, all strains showed very similar values. By day 5, ethanol yield under anaerobiosis was 0.38-0.40g/g. This value is lower than 0.45 g/g, typical value for anaerobic cultures, probably due to ethanol stripping through nitrogen flow. Ethanol yield in aerobiosis was 0.27-0.30g/g for the same sample point (Table 2). This value is significantly below that for anaerobic conditions (about 25% lower), indicating that a substantial fraction of sugar metabolism was diverted from ethanol to other carbon sinks, presumably through respiratory metabolism.

**Table 2. Consumed sugars and yields of main fermentation metabolites on consumed sugars on day 5 after inoculation, in sterilized natural must in anaerobic (10 VVH N₂) and in aerobic (10 VVH air) conditions of strains grown in bioreactors. Capital letters in the same row indicate statistical differences (p ≤ 0.05).**

| | | PR50 | | | PR117 | | | PR543 | | | PR1018 | | |
|---|---|---|---|---|---|---|---|---|---|---|---|---|---|
| | | mean | ± | std dev | mean | ± | std dev | mean | ± | std dev | mean | ± | std dev |
| % Consumed Sugars | N₂ | 83,34 | ± | 0,95 B | 43,84 | ± | 2,63 A | 96,49 | ± | 1,64 C | 81,93 | ± | 0,72 B |
| | Air | 77,70 | ± | 1,23 B | 69,20 | ± | 1,03 A | 77,37 | ± | 1,44 B | 90,40 | ± | 0,63 C |
| Glycerol Yield (mg/g) | N₂ | 52,09 | ± | 2,01 A | 79,54 | ± | 4,08 C | 55,62 | ± | 2,59 A | 65,47 | ± | 2,63 B |
| | Air | 32,39 | ± | 1,08 A | 50,96 | ± | 0,50 D | 40,75 | ± | 1,98 C | 37,64 | ± | 0,39 B |
| Acetic Acid Yield (mg/g) | N₂ | 0,22 | ± | 0,02 A | 0,24 | ± | 0,01 A | 0,58 | ± | 0,05 B | 0,41 | ± | 0,17 AB |
| | Air | 5,34 | ± | 0,65 B | 0,47 | ± | 0,02 A | 6,70 | ± | 1,47 B | 0,23 | ± | 0,01 A |
| Ethanol Yield (g/g) | N₂ | 0,39 | ± | 0,00 AB | 0,40 | ± | 0,00 AB | 0,40 | ± | 0,00 B | 0,38 | ± | 0,01 A |
| | Air | 0,27 | ± | 0,01 A | 0,28 | ± | 0,00 AB | 0,29 | ± | 0,00 B | 0,30 | ± | 0,01 C |

The response of sugar consumption to differences in oxygen availability was opposite for the two wild strains (PR117 and PR1018), showing faster sugar consumption under aerobic conditions; or the commercial strains (PR50 and PR543), which consumed it faster under anaerobic conditions (Figure 2). Strain PR117 consumed sugars more slowly than the others, and after 11 days residual sugars for this strain were 2.67g/L in aerobiosis but 22.48 g/L in anaerobiosis. Strains PR1018 and PR50 finished sugars in 7 days in anaerobiosis; whereas in aerobiosis PR1018 took only 6 days, and PR50 took longer than in anaerobiosis. Strain PR543 depleted sugars in 6 days in anaerobiosis. In aerobiosis sugar consumption was arrested after day 5, leaving more than 43g/L residual sugars. Slow sugar consumption precludes commercial application of strains PR543 and PR117 under fermentation conditions leading to reduced ethanol yield.

For each strain, the glycerol yield was higher in anaerobiosis than in aerobiosis (Table 2). In all cases, it decreased with time till sugar exhaustion (Figure 2). This is most probably due to glycerol being mostly produced during the first stages of fermentation under both aerobic and anaerobic conditions (Table 2).

Acetic acid yield under anaerobic conditions was low for all four strains over time (Figure 2). By day 5, two strains produced significantly higher amounts of acetic acid under aerobic conditions than under anaerobic conditions (Table 2). Strain PR1018 had a remarkably low acetic acid yield during the first days of aerobic fermentation, before sugars were depleted. Commercial strain PR50 showed high acetic acid yield under aerobic conditions, increasing moderately after sugar exhaustion. Strain PR543 had been chosen for this experiment because the low acetic acid yield in the screening in shake flasks. However, this feature could not be confirmed.

### 2.3. Volatile compounds produced under aerobic and anaerobic conditions

The profile of volatile compounds was rather different between aerobic and anaerobic conditions for all four strains (Figure 3). Compounds that were more abundant under aerobic conditions include carbonyl compounds and compounds containing a dioxane or dioxolane ring. Other groups of compounds did not respond homogenously to oxygenation, with compound concentrations varying in one direction or the other. There were also compounds showing a different response to aeration, depending on the yeast strain.

The production of acetaldehyde and its acetal derivatives was higher under aerobic conditions, but significantly lower for strain PR1018 than for the other three strains (Figure 3). The result was similar for acetoin and diacetyl. In contrast, this strain (PR1018) showed the highest levels of 2,3-butanediol among the four strains under aerobic conditions. Ethyl acetate levels also increased with aeration, as carbonyl compounds. The highest values for this compound were found for strain PR543 (Figure 3).

Three additional acetate esters were quantified from these fermentation experiments. Isobutyl acetate was detected only under aerobic conditions, and only for strains PR50, PR543 and PR1018 (Figure 3). Isoamyl acetate and phenylethyl acetate were more abundant under anaerobic conditions for all strains. The highest levels of the three esters under aerobic conditions were found for the PR1018 strain, and the lowest ones for the PR117 strain. PR543 was the strain showing the highest concentration of ethyl esters, both under aerobic and anaerobic fermentation conditions.

Isobutanol and linear alcohols showed their highest levels in aerobiosis, while isoamyl alcohols, phenylethanol, and methionol were mostly produced in anaerobiosis (Figure 3). Strain PR1018 showed the highest levels of 2-phenylethanol, an interesting aroma compound in wine, under both fermentation conditions.

In summary, the impact of oxygenation on the production of these families of compounds depended on the yeast strain and the specific compound, with instances of both higher and lower production under aerobic conditions. All detected compounds containing a dioxane or dioxolane ring clearly increased in response to aeration (Figure 3). Compounds of this family had been previously associated with the oxidation of wines. This increase was observed for all four strains, but the lowest levels under aerobic conditions were found for strain PR1018. This is in agreement with the lower levels of acetaldehyde also observed for this strain under the same fermentation conditions (Figure 3). Acetaldehyde is one of the compounds involved in the formation of dioxolane ring containing molecules, together with wine diols.

A Principal Component Analysis (PC) performed using the data in Figure 3 was performed. A total of 7 Principal Components were obtained. PC1 explained 46%, and PC2 a 19% of the variance. A representation of samples in the plane defined by PC1 and PC2 as variables shows that PC1 clearly separates aerobic from anaerobic samples (Figure 4). According to this plot, among aerobic cultures those of strain PR1018 are closer to anaerobic cultures than any other strain, mostly due to the low levels of acetaldehyde and related compounds, as well as dioxane/dioxolane ring molecules. Other molecules defining aerobic fermentations, like propanol and butanol are also lower for PR1118 aerobic cultures. These data also point to PR1018 as a strain especially useful for aerobic fermentations resulting in lower ethanol but similar sensory profile as standard winemaking conditions.

### 3. DISCUSSION

The phenotypic analysis under aerobic conditions of this collection of *S. cerevisiae* strains has shown a relative homogeneity of *S. cerevisiae* isolates in some fermentation parameters, with ethanol yield being the most homogeneous one. Indeed, ethanol yield in this species seems to be much more dependent on growth conditions than on the specific strain. However, acetic acid yield showed the highest diversity, with almost one order of magnitude between the extreme values.

Oxygenation of grape must during fermentation results in clearly reduced ethanol yields (about 25% lower for bioreactors flushed with air as compared to those flushed with nitrogen). This fact, combined with the identification of PR1018 *S. cerevisiae* strain, that shows very low volatile acidity during aerobic fermentation, opens the way to use *S. cerevisiae* for alcohol level reduction in wines.

The high impact of oxygen availability on yeast metabolism goes beyond respiration, ethanol yield, or acetic acid yield; and its influence on the profile of volatile compounds is especially relevant for winemaking. In the selection of novel *S. cerevisiae* wine yeasts, compatible with aerobic fermentation, it must be considered that differences in aeration regimes can lead to very significant differences in fermentation output. Nevertheless, the behaviour of PR1018, was consistent across different aerobic conditions, showing high promise for industrial application.

Furthermore, the volatile profile of PR1018 under aerobic conditions is the one falling closer to anaerobic conditions among the yeast strains tested. This suggest that, by using this strain as starter, the impact of aeration on the sensory features of wines might be minimized.

## Claims

1. A *Saccharomyces cerevisiae* strain with deposit number CECT 13195.

2. A strain derived from the strain according to claim 1.

3. The strain derived according to claim 2, wherein said strain is a genetically modified mutant.

4. The strain according to any one of claims 1 to 3, wherein said strain is in the form of viable cells.

5. A composition comprising the strain according to any of claims 1 to 4.

6. Composition according to claim 5, wherein it further comprises at least an additive.

7. Use of the strain according to any one of claims 1 to 4 or the composition according to claim 5 or 6 for fermented beverages production.

8. Use according to claim 7 wherein the fermented beverage is selected of the list consisting of: wine, beer, sparkling wine, sake and cider, preferably the fermented beverage is wine.

9. A method for fermented beverages production which comprises:
(i) inserting the strain according to any one of claims 1 to 4 and/or the composition according to claim 5 or 6 in a sugar-containing solution, and
(ii) incubating the sugar-containing solution obtained in (i) at a temperature between 8ºC and 35ºC during 2 to 10 days, under aerobic conditions, obtaining a partially or fully fermented sugar-containing solution,
wherein the sugar-containing solution of step (i) comprises a hexose content between 15-35% and the fermented sugar-containing solution obtained in step (ii) comprises a residual hexose content between 0 to 10%.

10. The method according to claim 9 wherein fermented beverage is selected of the list consisting of: wine, beer, sparkling wine, sake and cider, preferably is wine.

11. The method according to claims 9 or 10 wherein the sugar-containing solution is grape must.

12. The method according to any one of claims 9 to 11 wherein the temperature of step (ii) is 25ºC.

13. The method according to any one of claims 10 to 13 wherein the aerobic conditions of step (ii) comprise a constant gas flow with compressed air at 1 to 20 VVH, preferably is 10 VVH.

14. The method according to any one of claims 10 to 13 further comprises a step (iii) incubating the partially fermented sugar-containing solution obtained in (ii) under anaerobic conditions at a temperature between 8ºC and 35ºC, until sugar exhaustion.

## Amended claims

### Amended claims in accordance with Rule 137(2) EPC.

1. A *Saccharomyces cerevisiae* strain with deposit number CECT 13195.

2. The strain according to claim 1, wherein said strain is in the form of viable cells.

3. A composition comprising the strain according to any of claims 1 to 2.

4. Composition according to claim 3, wherein it further comprises at least an additive.

5. Use of the strain according to any one of claims 1 or 2 or the composition according to claim 3 or 4 for fermented beverages production.

6. Use according to claim 5 wherein the fermented beverage is selected of the list consisting of: wine, beer, sparkling wine, sake and cider, preferably the fermented beverage is wine.

7. A method for fermented beverages production which comprises:
(i) inserting the strain according to any one of claims 1 or 2 and/or the composition according to claim 3 or 4 in a sugar-containing solution, and
(ii) incubating the sugar-containing solution obtained in (i) at a temperature between 8°C and 35°C during 2 to 10 days, under aerobic conditions, obtaining a partially or fully fermented sugar-containing solution,
wherein the sugar-containing solution of step (i) comprises a hexose content between 15-35% and the fermented sugar-containing solution obtained in step (ii) comprises a residual hexose content between 0 to 10%.

8. The method according to claim 7 wherein fermented beverage is selected of the list consisting of: wine, beer, sparkling wine, sake and cider, preferably wine.

9. The method according to claims 7 or 8 wherein the sugar-containing solution is grape must.

10. The method according to any one of claims 7 to 9 wherein the temperature of step (ii) is 25°C.

11. The method according to any one of claims 7 to 10 wherein the aerobic conditions of step (ii) comprise a constant gas flow with compressed air at 1 to 20 VVH, preferably 10 VVH.

12. The method according to any one of claims 7 to 11 further comprising a step (iii) of incubating the partially fermented sugar-containing solution obtained in (ii) under anaerobic conditions at a temperature between 8°C and 35°C, until sugar exhaustion.
